# FASCICULE DE BREVET EUROPEEN

(11) **EP 4 072 451 B1**
(45) Date de publication et mention de la délivrance du brevet: **24.04.2024**
(21) Numéro de dépôt: 20842274.1
(22) Date de dépôt: 09.12.2020
(51) Int. Cl.: A61B 17/88

(54) **DISPOSITIF D'INJECTION ADAPTÉ POUR UNE INJECTION DE PARTICULES À L'INTÉRIEUR D'UNE CAVITÉ OSSEUSE**
INJEKTIONSVORRICHTUNG GEEIGNET ZUM EINSPRITZEN VON TEILCHEN IN EINEN KNOCHENHOHLRAUM
INJECTION DEVICE SUITABLE FOR INJECTING PARTICLES INTO A BONE CAVITY

(30) Priorité: 11.12.2019 FR 1914143
(43) Date de publication de la demande: 19.10.2022
(73) Titulaire: Spinedust, 13008 Marseille (FR)
(72) Inventeur: LEVRIER, Olivier, 13008 Marseille (FR); GRAZIANI, Noël, 13004 Marseille (FR)
(74) Mandataire: Germain Maureau
(86) Numéro de dépôt international: PCT/FR2020/052359
(87) Numéro de publication internationale: WO 2021/116605

(56) Documents cités:
- WO-A1-2019/014895
- CN-U- 209 074 849

## Description

L'invention concerne le domaine de l'orthopédie et a pour objets un dispositif d'injection pour une injection de particules à l'intérieur d'une cavité osseuse, ainsi qu'un kit d'injection comprenant un tel dispositif d'injection ainsi que des particules associées.

La vertébroplastie percutanée est une technique chirurgicale, peu invasive et guidée par imagerie aux rayons X qui consiste à injecter dans une vertèbre, au moyen d'une aiguille de biopsie osseuse et à partir du pédicule, une composition de ciment osseux qui va se durcir afin de traiter une fracture (par exemple dans le cadre d'une fracture sur lésion tumorale, d'angiomes agressifs ou de fractures vertébrales ostéoporotiques).

Les compositions de ciment osseux à base de polyméthyl-méthylacrylate (ci-après abrégé « PMMA ») sont habituellement utilisées pour ce type d'intervention chirurgicale.

Cependant, les compositions de ciment osseux à base de PMMA présentent les inconvénients suivants :
- la polymérisation du PMMA est une réaction exothermique importante qui peut endommager les tissus organiques environnants une fois l'injection de composition de ciment osseux réalisée ;
- les propriétés mécaniques du PMMA étant éloignées de celles de l'os, le ciment osseux peut l'endommager si le ciment osseux est trop rigide, en particulier dans le cas d'os ostéopénique ;
- les compositions de ciment osseux à base de PMMA ne possèdent pas d'activité *in vivo* pour la liaison osseuse. En d'autres termes, de telles compositions ne peuvent former de liaison chimique avec les tissus osseux humains ou ne peuvent être remplacées par des os nouvellement formés. C'est pourquoi, l'interface entre de telles compositions de ciment osseux et l'os peut être rompue à long terme, causant un risque de descellement de l'os.

Les documents CN 209 074 849 U et WO 2019/014895 A1 décrivent tous les deux des dispositifs d'injection de ciment osseux.

C'est pourquoi, au vu de ces inconvénients, la mise au point de solutions alternatives aux compositions de ciment osseux à base de PMMA serait tout à fait bénéfique pour les praticiens et les patients.

L'inventeurde la présente demande a mis au point un dispositif d'injection qui permet d'injecter des particules à l'intérieur d'une cavité osseuse, qui surmonte parfaitement les inconvénients liés aux compositions de ciment osseux à base de PMMA qui ont été rappelés ci-dessus.

L'invention a aussi pour objet un dispositif d'injection adapté pour une injection de particules à l'intérieur d'une cavité osseuse, ledit dispositif d'injection comprenant :
- un réservoir propre à contenir au moins en partie les particules ;
- une aiguille d'injection de forme tubulaire autour d'un axe principal et munie d'une extrémité proximale fixée sur une embase du réservoir et ouverte sur le réservoir, et d'une extrémité distale opposée à l'extrémité proximale et ouverte ; et
- une vis sans fin mobile en rotation autour de l'axe principal à l'intérieur de l'aiguille d'injection et s'étendant au moins depuis l'extrémité proximale jusqu'à au moins l'extrémité distale de l'aiguille d'injection ;
- un actionneur couplé à la vis sans fin pour l'entraîner en rotation.

Un tel dispositif d'injection est particulièrement adapté pour injecter les particules à travers l'aiguille d'injection en étant convoyées par la vis sans fin (aussi connue sous le nom de vis d'Archimède). L'emploi de la vis sans fin favorise le déplacement des particules, tout en limitant les risques d'agrégation des particules qui formerait alors un bouchon nuisible à l'injection. Une fois injectées, ces particules seront réparties dans la cavité osseuse et procureront une résistance mécanique élevée. A la différence des compositions de ciment osseux à base de PMMA qui sont non poreuses, les particules formeront un matériau poreux et granulaire qui présente ainsi une structure plus légère, plus élastique et plus résistante. En outre, ce matériau à base de particules s'intègre parfaitement au sein de l'os, étant donné que les vertèbres sont des matériaux poreux.

Selon une possibilité, l'actionneur comprend un arbre couplé en rotation à la vis sans fin, ledit arbre sortant du réservoir pour être accouplé à un moteur.

Selon une autre possibilité, un piston est monté coulissant dans le réservoir et mobile en translation selon l'axe principal. Un tel piston n'a pas vocation à pousser les particules (ou une composition de ciment osseux comprenant de telles particules en solution dans une composition liquide ou gel), dans la mesure où l'injection est effectuée par la vis sans fin rotative. En effet, il est préférable d'éviter d'appliquer une pression forte en partie haute du réservoir qui causerait une accumulation des particules.

De manière avantageuse, le dispositif d'injection comprend au moins un moyen de dispersion propre à disperser et mettre en suspension les particules dans une composition liquide ou gel, par exemple de type colle biologique ou gel polymérique, à l'intérieur du réservoir. Le ou les moyens de dispersion ont pour objet de fluidifier la composition de ciment osseux (qui comprend comprenant les particules en solution dans la composition liquide ou gel) pour un écoulement fluide à travers l'aiguille d'injection, en dispersant les particules pour éviter des agrégations et des sédimentations de ces particules.

Dans une réalisation particulière, le moyen de dispersion est choisi parmi les moyens suivants ou une combinaison de tout ou partie des moyens suivants :
- un système d'introduction pour une introduction de la composition liquide ou gel à l'intérieur du réservoir, ledit système d'introduction comprenant un moyen de mise en circulation situé à l'extérieur du réservoir et assurant une circulation de la composition liquide ou gel dans un ou plusieurs conduits d'introduction reliés à un ou plusieurs points d'introduction débouchant dans l'embase du réservoir ;
- un système vibrateur pour appliquer une vibration sur le réservoir ou sur l'aiguille d'injection ;
- un système de brassage pour brasser les particules en solution dans la composition liquide ou gel à l'intérieur du réservoir ;
- un système oscillant comprenant un piston monté coulissant dans le réservoir et mobile en translation selon l'axe principal, et un actionneur oscillant propre à imprimer un mouvement d'oscillation au piston selon l'axe principal.

Le système d'introduction permet d'introduire la composition liquide ou gel au niveau de l'embase (en partie basse du réservoir), générant ainsi un flux liquide susceptible de décoller les particules de l'embase et de disperser les particules au niveau de l'extrémité proximale de l'aiguille d'injection, et donc en entrée de la vis sans fin.

Ce système d'introduction peut être complété d'un système de recirculation pour une recirculation de la composition liquide ou gel, qui comprendrait un ou plusieurs points d'évacuation débouchant dans le réservoir (préférentiellement en partie haute du réservoir) et reliés à un ou plusieurs conduits d'évacuation pour un retour de la composition liquide ou gel au niveau du moyen de mise en circulation. Ainsi, de la composition liquide ou gel entre au niveau du ou des points d'introduction, et de la composition liquide ou gel sort au niveau du ou des points d'évacuation, le moyen de mise en circulation (comme par exemple une pompe) assurant la circulation de la composition liquide ou gel entre le ou les points d'évacuation et le ou les points d'introduction.

Le système vibrateur applique une vibration qui permet de casser les amas de particules, dans le réservoir et/ou dans l'aiguille d'injection (et donc à l'intérieur de la vis sans fin). Le système vibrateur applique par exemple une vibration à une fréquence comprise entre 0,5 Hz et 10 KHz. Le système vibrateur est par exemple un système mécanique relié à un moteur et en contact avec le réservoir et/ou l'aiguille d'injection.

Le système de brassage applique un mouvement mécanique de brassage à l'intérieur du réservoir, et il peut comprendre un brasseur (comme par exemple un agitateur ou une pale) accouplé à un moteur situé à l'extérieur du réservoir. Ce système de brassage permet ainsi de casser les amas de particules et donc de fluidifier la composition de ciment osseux.

Le système oscillant applique un mouvement d'oscillation au piston selon l'axe principal, ce qui revient à un mouvement oscillant de bas en haut en partie haute du réservoir, qui procure un gradient de pression qui participe à une dispersion des particules à l'intérieur du réservoir.

Dans un mode de réalisation particulier, la vis sans fin est de forme générale cylindrique ou de forme générale tronconique. L'utilisation d'une vis sans fin tronconique diminue le volume local des particules (le cas échéant de la composition de ciment osseux) et oblige les particules à se réorganiser, augmentant ainsi le taux de partage en milieu granulaire. En rhéologie granulaire, un taux de partage plus élevé entraîne un coefficient de frottement plus élevé entre les particules et la contrainte solide. Une vis sans fin cylindrique limite quant à elle le taux de partage dans le milieu granulaire.

Selon une possibilité, la vis sans fin s'étend intégralement à l'intérieur de l'aiguille d'injection, en s'étendant depuis l'extrémité proximale jusqu'à l'extrémité distale de l'aiguille d'injection.

Selon une autre possibilité, la vis sans fin s'étend à l'intérieur de l'aiguille d'injection et aussi à l'intérieur du réservoir, en s'étendant au-delà de l'extrémité proximale de l'aiguille d'injection, de sorte que la vis sans fin présente au moins deux parties successives comprenant :
- une partie inférieure qui s'étend depuis l'extrémité proximale jusqu'à l'extrémité distale de l'aiguille d'injection ;
- une partie supérieure qui prolonge la partie inférieure au-delà de l'extrémité proximale et qui s'étend à l'intérieur du réservoir.

Dans une réalisation avantageuse, la vis sans fin est ajustable en translation le long de l'axe principal, dans le but avantageux de permettre une impaction des particules à l'intérieur de la cavité osseuse au moyen de l'extrémité distale de la vis sans fin.

Une telle possibilité d'ajustement permet ainsi de rétracter la vis sans fin à l'intérieur de l'aiguille d'injection, c'est-à-dire sans dépasser de l'extrémité distale de l'aiguille d'injection, et de déployer la vis sans fin hors de l'aiguille d'injection, en la faisant dépasser au-delà de l'extrémité distale de l'aiguille d'injection afin de pouvoir plonger à l'intérieur de la cavité osseuse, dans le but de favoriser une impaction des particules à l'intérieur de la cavité osseuse. La progression de la vis sans fin dans la cavité osseuse, et donc hors de l'aiguille d'injection, pourra avantageusement être contrôlée de façon radiologique, et un verrouillage de la position de déploiement optimale de la vis sans fin sera effectué.

Dans un mode de réalisation particulier, le dispositif d'injection comprend en outre un implant expansible comprenant au moins un cathéter et une enveloppe expansible configurable entre :
- un état contracté dans lequel l'enveloppe expansible est contrainte radialement dans le cathéter pour une délivrance de l'enveloppe expansible à l'intérieur de la cavité osseuse, et
- un état déployé dans lequel l'enveloppe expansible est étendue hors du cathéter avec une configuration en forme de poche pour être remplie en particules.

Une telle enveloppe expansible est ainsi prévue pour être déployée à l'intérieur de la cavité osseuse, et ainsi former une poche de contention à l'intérieur de la cavité osseuse, et ensuite les particules (seules ou en solution dans une composition liquide ou gel) seront injectées à l'intérieur de cette enveloppe expansible qui procurera une contention des particules. Une telle enveloppe expansible permettra également d'éviter une migration secondaire des particules dans la circulation veineuse sanguine. L'emploi d'une telle enveloppe expansible permet éventuellement de se passer d'une composition liquide ou gel et d'injecter les particules directement et seules ; et dans ce cas c'est cette enveloppe expansible qui assurera la fonction de contention.

Selon une possibilité, l'enveloppe expansible comporte au moins une pluralité de fils agencés pour former ensemble un maillage, ou l'enveloppe expansible est une enveloppe pleine réalisée dans un matériau élastique ou gonflable.

Selon une autre possibilité, le cathéter traverse la vis sans fin ; la vis sans fin étant alors munie d'un orifice longiligne dans sa longueur, à travers lequel est inséré le cathéter pour atteindre la cavité osseuse et ensuite déployer l'enveloppe expansible à l'intérieur de la cavité osseuse.

L'invention a également pour objet un kit d'injection qui comprend au moins :
- un dispositif d'injection telle que décrit ci-dessus ; et
- des particules adaptées pour être injectées à l'intérieur d'une cavité osseuse au moyen dudit dispositif d'injection.

De manière avantageuse, ce kit d'injection comprend en outre un vibrateur osseux propre à appliquer une vibration sur un os durant une injection des particules à l'intérieur d'une cavité osseuse de l'os. Ainsi, ce vibrateur osseux va appliquer une vibration sur l'os, évitant ainsi une agrégation des particules à l'intérieur de la cavité osseuse qui conduirait à la formation d'espaces vides et donc à un remplissage partiel non recherché.

De manière encore avantageuse, la vis sans fin présente un pas de vis supérieur ou égal à une taille maximale des particules de la composition de ciment osseux.

Avantageusement, les particules sont choisies parmi des particules d'hydroxyapatite, d'acide poly(lactique-glycolique) (ci-après abrégé PLGA), d'amidon ou de chitosane.

Dans une réalisation particulière, les particules sont en solution dans une composition liquide ou gel pour former ensemble une composition de ciment osseux, où ladite composition liquide ou gel comporte un solvant liquide ou gel apte à diffuser à travers l'os.

La composition liquide ou gel a son solvant qui est à l'état liquide ou gel avant l'injection de la composition de ciment osseux, et les particules y sont réparties de manière homogène. Lorsque la composition de ciment osseux a été injectée dans une cavité osseuse, le solvant que contient la composition liquide ou gel va se diffuser dans le corps du patient. Du fait de la diffusion du solvant, cela va induire une densification des particules qui seront réparties de manière homogène à l'intérieur de la cavité osseuse.

Selon une première possibilité, la composition liquide ou gel est un gel polymérique comprenant un polymère en solution dans un solvant, ledit polymère étant choisi parmi :
- le polyéthylène glycol, l'acide hyaluronique ou l'hydroxypropyl methyl cellulose en solution dans de l'eau ;
- l'éthylène alcool vinylique ou l'acétate de cellulose en solution dans le diméthylsulfoxyde ;
- l'éthyl cellulose en solution dans l'éthanol.

Selon une seconde possibilité, la composition liquide ou gel est une colle biologique qui est choisie parmi les colles biologiques à base de :
- fibrinogène ou thrombine, le fibrinogène et la thrombine étant optionnellement en association avec de l'aprotinine,
- fibrine autologue,
- collagène, ou
- N-butyl-cyano acrylate.

La colle biologique et le gel polymérique décrits ci-dessus contiennent chacun un solvant apte à diffuser à travers l'os. Ils sont à l'état liquide avant l'injection de la composition de ciment osseux et contiennent en outre les particules qui y sont réparties de manière homogène. Lorsque la composition de ciment osseux a été injectée dans une cavité osseuse, le solvant que contient la colle biologique ou le gel polymérique va se diffuser dans le corps du patient. Du fait de la diffusion du solvant, cela va induire la solidification du gel ou de la colle biologique au sein duquel ou de laquelle seront emprisonnées les particules qui y seront réparties de manière homogène.

La composition de ciment osseux, à base de la colle biologique ou du gel polymérique décrits ci-dessus, présente ainsi les avantages suivants :
- être à l'état liquide avant son injection, ce qui facilite son injection et permet une répartition homogène des particules ;
- les particules sont amenées à l'intérieur de l'os et y demeurent reparties de manière homogène grâce à leur emprisonnement au sein du gel ou de la colle biologique qui a solidifié du fait de la diffusion du solvant dans le corps du patient.

La solidification au sein de l'os du gel polymérique ou de la colle biologique emprisonnant les particules permet d'obtenir un matériau dont les propriétés mécaniques sont proches de celles de l'os du patient. Ces propriétés mécaniques sont meilleures que celles des compositions de ciment osseux à base de PMMA. En effet, les particules que contient la composition de ciment osseux contribuent à augmenter la résistance mécanique de ladite composition. C'est pourquoi, la résistance mécanique de la composition de ciment osseux est supérieure à celle de composition à base de PMMA.

A la différence des compositions de ciment osseux à base de PMMA qui sont non poreuses, la composition de ciment osseux selon l'invention présente l'avantage d'obtenir après son injection et la solidification du gel polymérique ou de la colle biologique, un matériau poreux et granulaire qui présente ainsi une structure plus légère, plus élastique et plus résistante. En outre, ce matériau s'intègre parfaitement au sein de l'os, étant donné que les vertèbres sont des matériaux poreux.

La similarité des propriétés mécaniques avec celles de l'os du matériau poreux et granulaire ainsi obtenu à partir de l'injection de la composition de ciment osseux selon l'invention présente également l'avantage de rendre moins traumatique ladite injection.

La composition de ciment osseux selon l'invention présente en outre l'avantage d'induire la formation *in vivo* de cellules osseuses et donc la formation d'os.

A la différence des compositions de ciment osseux à base de PMMA, il n'y a pas de polymérisation *in vivo* de ladite composition de ciment osseux selon l'invention. Cela évite tous les problèmes liés aux réactions exothermiques de la polymérisation qui ont été mentionnés ci-dessus. En effet, dans la composition de ciment osseux selon l'invention, tous ses constituants sont chimiquement stables avant l'injection dans l'os du patient. Il n'y a donc pas de risque de réaction chimique dommageable au sein de l'os.

Les particules peuvent être de forme sphérique ou irrégulière.

En ce qui concerne les particules d'hydroxyapatite, leur taille peut être très variée. Par exemple, leur taille peut être comprise entre 15 et 45 µm ou entre 15 et 125 µm ou entre 25 et 75 µm ou entre 70 et 210 µm ou entre 32 et 106 µm ou entre 45 et 130 µm ou entre 160 et 400 µm ou entre 400 et 1000 µm ou entre 1000 et 2000 µm. Les particules peuvent être un mélange de particules dont la taille peut être choisie parmi au moins deux de ces intervalles de taille. Par exemple, les particules peuvent être un mélange de particules de tailles différentes, et par exemple de tailles comprises entre 15 et 125 µm, entre 160 et 400 µm et entre 400 et 1000 µm.

En ce qui concerne les particules de PLGA, leur taille peut être comprise entre 5 et 250 µm. Optionnellement, les particules de PLGA encapsulent au moins une substance active. Il peut s'agir d'une substance active antibiotique ou anticancéreuse.

En ce qui concerne les particules d'amidon, leur taille peut être comprise entre 50 et 200µm.

De manière générale, les particules présentent avantageusement une taille inférieure à 2000 µm.

Avantageusement, la composition liquide ou gel comprend un agent radio-opaque.

L'agent radio-opaque peut être une poudre métallique micronisée. Il peut s'agir d'une poudre de tantale, tungstène, iode minéral, baryum ou bismuth. En variante, l'agent radio-opaque peut être une solution de gadolinium d'acide éthylènediaminetétracétique.

L'agent radio-opaque peut être aussi une huile éthiodisée.

L'agent radio-opaque permet de visualiser en temps réel la localisation de l'injection de la composition de ciment osseux selon l'invention au sein de l'os.

Dans la composition de ciment osseux, le ratio du pourcentage massique de la composition liquide ou gel (gel polymérique ou la colle biologique) sur le pourcentage massique de particules est d'au maximum 1 : 4. Cela permet ainsi d'obtenir une pâte qui peut être injectée dans l'os avec le dispositif d'injection selon l'invention tel que décrit précédemment. La pâte peut être plus ou moins dense en particules. Ainsi, si ledit ratio est compris entre 1 : 4 et 1 : 9, la pâte de composition de ciment osseux se présente sous la forme d'un agrégat peu dense. Si ledit ratio est compris entre 1 : 9 et 1 : 99, la pâte de composition de ciment osseux se présente sous la forme d'un agrégat dense.

Ce ratio peut être aisément adapté en fonction de l'âge et de l'état de santé du patient dans lequel est injectée la composition de ciment osseux selon l'invention. En effet, ce ratio sera différent selon que le patient est une personne présentant une ostéopénie ou une ostéoporose ou une personne présentant une fracture vertébrale. De par ses constituants essentiels que sont d'une part un gel polymérique ou une colle biologique et d'autre part des particules, la composition de ciment osseux selon l'invention présente ainsi l'avantage d'être modulable en fonction des besoins spécifiques du patient pour lequel est injectée ladite composition. Selon les patients, une composition de ciment osseux selon l'invention plus ou moins dense sera injectée.

La composition de ciment osseux peut en outre comprendre des fibres. La longueur des fibres peut être comprise entre 5 µm et 5 mm. Le ratio entre la longueur des fibres et leur diamètre moyen peut être compris entre 2 et 1000. Les fibres permettent de renforcer le matériau résultant de la solidification du gel polymérique ou de la colle biologique emprisonnant des particules.

Ces fibres peuvent par exemple être des fibres d'origine naturelle, et notamment d'origine animale comme des fibres de soie ou d'origine végétale comme des fibres de cellulose. Ces fibres peuvent en variante être des fibres synthétiques, comme par exemple des fibres de chitosane, des fibres d'acide polylactique (aussi abrégé PLA), des fibres d'acide poly(lactique-glycolique) (aussi abrégé PLGA), des fibres de carbone. Dans une autre variante, ces fibres peuvent être un mélange de fibres, et par exemple un mélange de fibres d'origine naturelle et de fibres synthétiques.

L'invention sera mieux comprise à l'aide de la description détaillée qui est exposée ci-dessous en référence aux figures annexées représentant, à titre d'exemple non limitatif, plusieurs formes de réalisation d'un dispositif d'injection selon l'invention, ainsi qu'une illustration d'un implant expansible adapté à l'invention.
[Fig. 1] est une illustration schématique d'un kit d'injection selon un premier exemple de réalisation de l'invention, comprenant un premier dispositif d'injection et une composition de ciment osseux, où le premier dispositif d'injection est dans une première configuration.
[Fig. 2] est une illustration schématique du kit d'injection de la Figure 1, où le premier dispositif d'injection est dans une seconde configuration.
[Fig. 3] est une illustration schématique d'un kit d'injection selon un deuxième exemple de réalisation de l'invention, comprenant un deuxième dispositif d'injection et une composition de ciment osseux.
[Fig. 4] est une illustration schématique d'un implant expansible adapté à l'invention, lors d'étapes successives (a) à (g) d'insertion de son enveloppe expansible à l'intérieur d'une cavité osseuse.

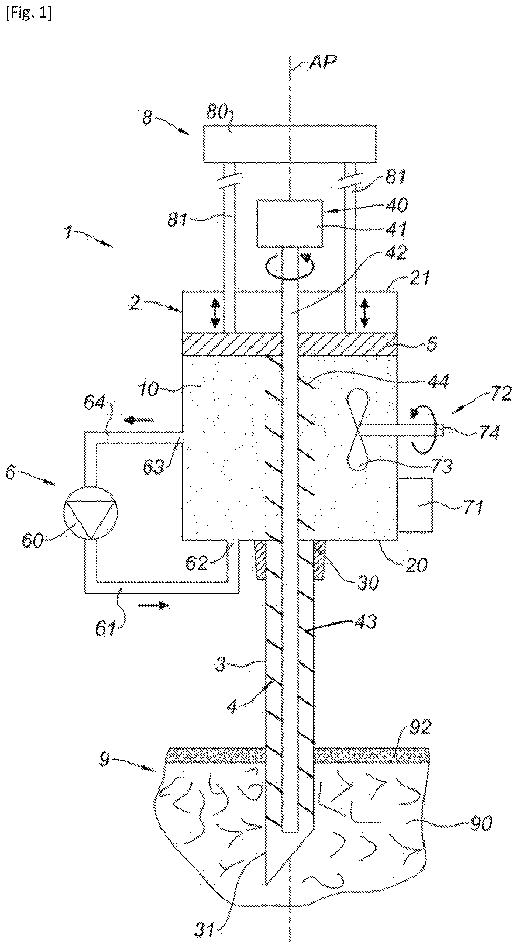

En référence aux Figures 1 à 3, un kit d'injection comprend des particules et un dispositif d'injection 1 adapté pour une injection des particules à l'intérieur d'une cavité osseuse 90 d'un os 9. Pour la suite de la description, en ce qui concerne le dispositif d'injection 1, des mêmes références numériques seront employées pour référencer des composants ou éléments identiques, similaires ou ayant des fonctions identiques.

Dans une réalisation avantageuse, les particules sont en solution dans une composition liquide ou gel pour former ensemble une composition de ciment osseux 10, où cette composition liquide ou gel comporte un solvant liquide ou gel apte à diffuser à travers l'os 9.

La composition de ciment osseux 10 comprend donc au moins les particules en solution dans la composition liquide ou gel qui est une colle biologique ou un gel polymérique, avec un ratio du pourcentage massique du gel polymérique ou de la colle biologique sur le pourcentage massique de particules qui est d'au maximum 1 : 4.

Les particules sont choisies parmi des particules d'hydroxyapatite, d'acide poly(lactique-glycolique) (ci-après abrégé PLGA), d'amidon ou de chitosane.

Le gel polymérique comprend un polymère en solution dans un solvant, où le polymère est choisi parmi :
- le polyéthylène glycol, l'acide hyaluronique ou l'hydroxypropyl methyl cellulose en solution dans de l'eau ;
- l'éthylène alcool vinylique ou l'acétate de cellulose en solution dans le diméthylsulfoxyde ;
- l'éthyl cellulose en solution dans l'éthanol.

La colle biologique est choisie parmi les colles biologiques à base de :
- fibrinogène ou thrombine, le fibrinogène et la thrombine étant optionnellement en association avec de l'aprotinine,
- fibrine autologue,
- collagène ou
- N-butyl-cyano acrylate.

La composition de ciment osseux 10 comprend optionnellement un agent radio-opaque tel qu'une poudre métallique micronisée choisie parmi les poudres de tantale, tungstène, baryum, bismuth, ou iode minéral. En variante, l'agent radio-opaque peut être une solution de gadolinium d'acide éthylènediaminetétracétique ou une huile éthiodisée.

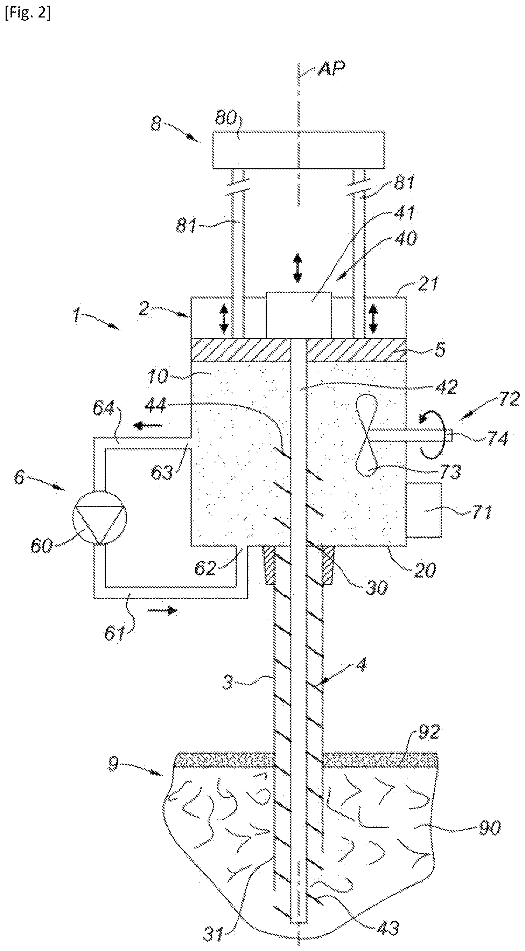

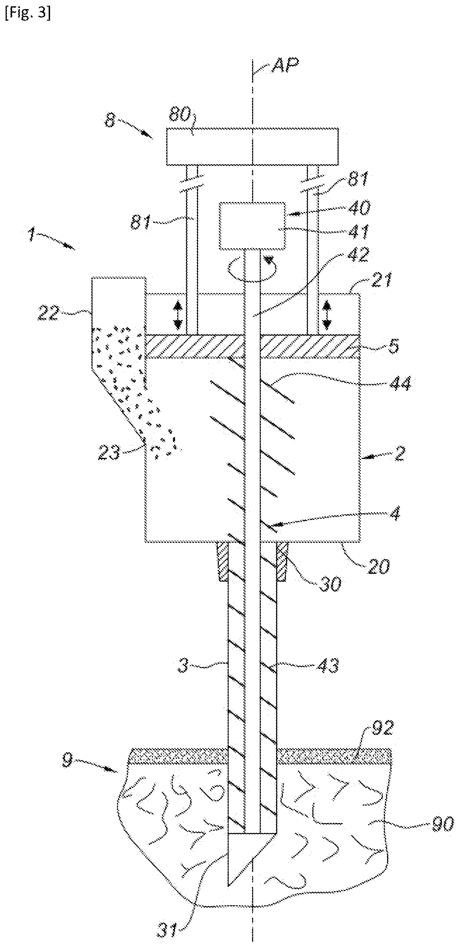

La suite de la description porte sur le dispositif d'injection 1, que ce soit dans le premier mode de réalisation illustré sur les Figures 1 et 2 ou dans le deuxième mode de réalisation illustré sur la Figure 3. Le dispositif d'injection 1 comprend un réservoir 2 propre à contenir la composition de ciment osseux 10, où ce réservoir 2 présente en partie basse une embase 20 (formant une paroi de fond) et présente en partie haute un sommet 21 (ouvert ou fermé par un couvercle amovible ou non). Le réservoir 2 peut être de forme générale cylindrique.

Dans l'exemple de la Figure 3, le dispositif d'injection 1 comprend également un réservoir annexe 22 qui est fixé au réservoir 2 et qui reçoit intérieurement des particules, et en particulier des particules en vrac, où ces particules sont celles employées pour former la composition de ciment osseux 10. Ce réservoir annexe 22 est en communication avec le réservoir 2 via une ouverture 23, et par exemple une ouverture munie d'une trappe configurable entre une position ouverte (pour une ouverture de la communication) et une position fermée (pour une fermeture de la communication). Ainsi, les particules peuvent être introduites au fur et à mesure dans le réservoir 2. Il est envisageable que ce réservoir 2 soit préalablement rempli (et éventuellement alimenté en continu) en colle biologique ou gel polymérique, tout comme il est envisageable que ce réservoir 2 contienne uniquement les particules au sec dans le cas où les particules sont introduites sans composition liquide ou gel.

Le dispositif d'injection 1 comprend une aiguille d'injection 3 de forme tubulaire autour d'un axe principal AP et munie d'une extrémité proximale 30 fixée sur l'embase 20 du réservoir 2 (par exemple au moyen d'une douille de connexion) et ouverte sur le réservoir 20, et d'une extrémité distale 31 opposée à l'extrémité proximale 30 et ouverte. L'extrémité proximale 30 est ainsi en liaison fluidique avec l'intérieur du réservoir 20, et l'extrémité distale 31 est prévue pour pénétrer à l'intérieur de la cavité osseuse 90. L'extrémité distale 31 peut être munie d'une terminaison en pointe ou biseautée, pour percer la couche externe 92 compacte et dure de l'os 9 ; optionnellement après un pré-perçage de cette couche externe 92.

Le dispositif d'injection 1 comprend une vis sans fin 4 mobile en rotation autour de l'axe principal AP à l'intérieur de l'aiguille d'injection 3 et s'étendant au moins depuis l'extrémité proximale 30 jusqu'à au moins l'extrémité distale 31 de l'aiguille d'injection 3. Cette vis sans fin 4 est ainsi montée à l'intérieur d'un canal interne de l'aiguille d'injection 3 ; ce canal interne étant de forme générale cylindrique. La vis sans fin 4 est de forme générale cylindrique (comme illustré dans la Figure 1) ou de forme générale tronconique (dans une variante non illustrée). Cette vis sans fin 4 présente un diamètre externe maximal qui est sensiblement inférieur à un diamètre interne du canal interne de l'aiguille d'injection 3, pour un montage quasiment sans jeu de la vis sans fin 4 dans le canal interne de l'aiguille d'injection 3 ; un jeu étant prévu pour autoriser la vis sans fin 4 à tourner à l'intérieur du canal interne de l'aiguille d'injection 3.

La vis sans fin 4 présente un pas de vis supérieur ou égal à une taille maximale des particules de la composition de ciment osseux 10, et par exemple un pas de vis égal ou supérieur à 2 millimètres.

Dans un mode de réalisation non illustré, la vis sans fin 4 s'étend depuis l'extrémité proximale 30 jusqu'à l'extrémité distale 31 de l'aiguille d'injection 3, et présente ainsi une longueur (distance mesurée le long de l'axe de rotation de la vis sans fin 4) sensiblement équivalente à la longueur de l'aiguille d'injection 3.

Dans les modes de réalisation des Figures 1 et 2 et de la Figure 3, la vis sans fin 4 s'étend depuis l'extrémité proximale 30 jusqu'à l'extrémité distale 31 de l'aiguille d'injection 3, et s'étend aussi au-delà de l'extrémité proximale 30 de sorte que cette vis sans fin 4 s'étend en partie à l'intérieur du réservoir 2. Autrement dit, la vis sans fin 4 présente deux parties successives :
- une partie inférieure 43 qui s'étend depuis l'extrémité proximale 30 jusqu'à l'extrémité distale 31 de l'aiguille d'injection 3, et présente ainsi une longueur sensiblement équivalente à la longueur de l'aiguille d'injection 3 ;
- une partie supérieure 44 qui prolonge la partie inférieure 43 et qui s'étend à l'intérieur du réservoir 2.

Par ailleurs, la vis sans fin 4 est ajustable en translation le long de l'axe principal AP, et cette vis sans fin 4 est ainsi configurable entre :
- une première configuration, visible sur la Figure 1, dans laquelle la vis sans fin 4 est rétractée à l'intérieur de l'aiguille d'injection 3, dans le sens où cette vis sans fin 4 ne dépasse pas de l'extrémité distale 31 de l'aiguille d'injection 3 ; et
- une seconde configuration, visible sur la Figure 2, dans laquelle la vis sans fin 4 est déployée hors de l'aiguille d'injection 3, dans le sens où cette vis sans fin 4 dépasse de l'extrémité distale 31 de l'aiguille d'injection 3 pour plonger à l'intérieur de la cavité osseuse 90.

De préférence, cet ajustement est maîtrisé dans le sens où la longueur de dépassement de la vis sans fin 4, au-delà de l'extrémité distale 31 de l'aiguille d'injection 3, est maîtrisé et verrouillable, ce qui signifie que le chirurgien peut ajuster et bloquer cette longueur de dépassement.

Bien que non illustré, il est également envisageable que la vis sans fin 4 de la Figure 3 soit également ajustable en translation le long de l'axe principal AP.

La vis sans fin 4 a pour fonction de guider et convoyer les particules de la composition de ciment osseux 10 depuis le réservoir 2 jusqu'à l'extrémité distale 31 de l'aiguille d'injection 3, et donc jusqu'à la cavité osseuse 90 de l'os 9. Dans l'exemple de la Figure 1, ce guidage et ce convoyage des particules par la vis sans fin 4 s'opèrent à partir de l'extrémité proximale 30 de l'aiguille sans fin 3, donc à partir de l'embase 20. Dans l'exemple de la Figure 2, ce guidage et ce convoyage des particules par la vis sans fin 4 s'opèrent depuis l'intérieur du réservoir 2, au moyen de la partie supérieure 44 de la vis sans fin 4 qui commence à convoyer les particules tout en prodiguant un brassage du fait de la rotation de cette partie supérieure 44 dans le réservoir 2 qui génère un flux tourbillonnant.

Dans le mode de réalisation de la Figure 3, la partie supérieure 44 de la vis sans fin 40 peut avoir un diamètre supérieur à celui de la partie inférieure 43, et il est notamment possible que cette partie supérieure 44 présente un diamètre croissant depuis la partie inférieure 43. Dans le mode de réalisation des Figures 1 et 2, la partie supérieure 44 de la vis sans fin 40 a le même diamètre que la partie inférieure 43.

Le dispositif d'injection 1 comprend un actionneur 40 couplé à la vis sans fin 4 pour l'entraîner en rotation. Dans l'exemple non limitatif illustré en Figure 1, l'actionneur 40 comprend un moteur 41 accouplé à un arbre 42, où cet arbre 42 est couplé en rotation à la vis sans fin 4, et notamment l'arbre 42 présente une portion basse autour de laquelle est fixée la vis sans fin 4. L'arbre 42 sort du réservoir 2 pour être accouplé au moteur 41 disposé à l'extérieur du réservoir 2.

Dans le cas où la vis sans fin 4 est ajustable en translation le long de l'axe principal AP, alors l'actionneur 40, et donc le moteur 41 et l'arbre 42, sont solidaires en translation avec la vis sans fin 4.

Le dispositif d'injection 1 comprend un piston 5 qui est monté coulissant dans le réservoir 2 et mobile en translation selon l'axe principal AP. Ce piston 5 est traversé de manière étanche par l'arbre 42.

Il est envisageable que ce piston 5 soit vissé sur le pourtour de l'arbre 42, qui permet une avancée du piston 5 proportionnellement au volume évacué par la vis sans fin 4 à l'intérieur de la cavité osseuse 90. Dans ce cas, le piston 5 doit pouvoir tourner et ainsi l'actionneur 40 doit être adapté pour que le piston 5 puisse se déplacer en spirale.

Le dispositif d'injection 1 comprend également un ou plusieurs moyens de dispersion propres à disperser et mettre en suspension les particules de la composition de ciment osseux 10 dans la colle biologique ou le gel polymérique à l'intérieur du réservoir 2.

Un premier moyen de dispersion est un système d'introduction 6 pour une introduction de colle biologique ou de gel polymérique à l'intérieur du réservoir 2, où ce système d'introduction 6 comprend un moyen de mise en circulation 60 (comme par exemple une pompe fluidique) situé à l'extérieur du réservoir 2 et assurant une circulation de colle biologique ou de gel polymérique dans un ou plusieurs conduits d'introduction 61 reliés à un ou plusieurs points d'introduction 62 débouchant dans l'embase 20 du réservoir 2.

Ce système d'introduction 6 peut par exemple être complété d'un système de recirculation pour une recirculation de la colle biologique ou du gel polymérique, qui comprend un ou plusieurs points d'évacuation 63 débouchant dans le réservoir (préférentiellement en partie haute du réservoir 2) et reliés à un ou plusieurs conduits d'évacuation 64 pour un retour de la colle biologique ou du gel polymérique au niveau du moyen de mise en circulation 60. Ainsi, de la colle biologique ou du gel polymérique entre au niveau du ou des points d'introduction 62, et de la colle biologique ou du gel polymérique sort au niveau du ou des points d'évacuation 63, le moyen de mise en circulation 60 assurant la circulation de la colle biologique ou du gel polymérique dans le ou les conduits d'introduction 61 et dans le ou les conduits d'évacuation 64, entre le ou les points d'évacuation 63 et le ou les points d'introduction 62. Le ou les points d'évacuation 63 et le ou les points d'introduction 62 peuvent être munis de filtres pour éviter le passage des particules à l'intérieur des conduits 63, 64.

Un deuxième moyen de dispersion est un système vibrateur 71 pour appliquer une vibration sur le réservoir 2 ou sur l'aiguille d'injection 3, comme par exemple une vibration à une fréquence comprise entre 0,5 Hz et 10 KHz.

Un troisième moyen de dispersion est un système de brassage 72 pour brasser la composition de ciment osseux 10 à l'intérieur du réservoir 2, et il peut comprendre un brasseur 73 accouplé à un moteur (non illustré) situé à l'extérieur du réservoir 2 via un arbre d'accouplement 74 qui traverse une paroi du réservoir 2 ou qui traverse le piston 5.

Un quatrième moyen de dispersion est un système oscillant 8 comprenant le piston 5 et comprenant un actionneur oscillant 80 propre à imprimer un mouvement d'oscillation au piston 5 selon l'axe principal via un ou plusieurs éléments de transmission 81 qui transmettent un mouvement oscillant généré par l'actionneur oscillant 90 à destination du piston 5.

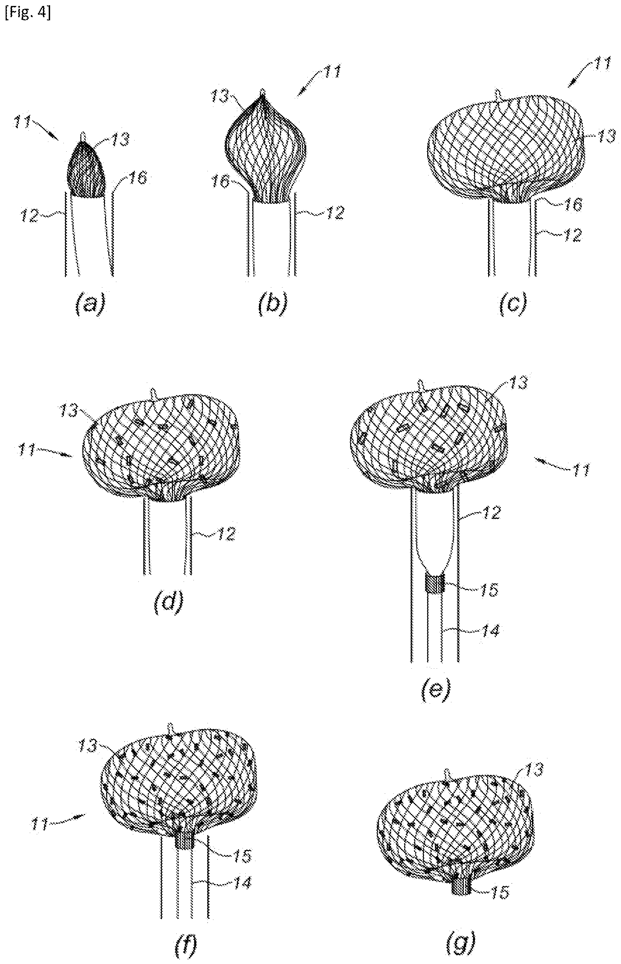

En référence à la Figure 4, il est envisageable de prévoir que le dispositif d'injection 1 comprenne en outre un implant expansible 11 comprenant au moins un cathéter 12 et une enveloppe expansible 13 configurable entre :
- un état contracté (visible sur la Figure 4(a)) dans lequel l'enveloppe expansible 13 est contrainte radialement dans le cathéter 12 pour une délivrance de l'enveloppe expansible 13 à l'intérieur de la cavité osseuse, et
- un état déployé (visible sur la Figures 4(c) à 4(g)) dans lequel l'enveloppe expansible 13 est étendue hors du cathéter 12 avec une configuration en forme de poche.

Cet implant expansible 11 comprend ainsi un organe de poussée tubulaire 14 (visible sur les Figures 4(e) et 4(f)) formé d'une tige tubulaire coulissant à l'intérieur du cathéter 12 et raccordée par une bague 15 à l'enveloppe expansible 13, de sorte que les particules (visibles sur les Figures 4(d) à 4(g)) pourront être injectées à l'intérieur de l'enveloppe expansible 13 à travers cet organe de poussée tubulaire 14.

Le cathéter 12 est muni d'une extrémité proximale 16 qui débouche dans la cavité osseuse, et l'enveloppe expansible 13 se déploie en sortant de cette extrémité proximale 16.

Bien que non illustré, le cathéter 12 traverse la vis sans fin 4, et traverse donc l'arbre 42, selon l'axe principal AP, et l'organe de poussée tubulaire 14 présente une ouverture en communication avec le réservoir 2 pour permettre l'injection des particules à l'intérieur de l'enveloppe expansible 13 en passant à l'intérieur de l'organe de poussée tubulaire 14.

Dans une première réalisation, l'enveloppe expansible 13 est une enveloppe auto-expansible maillée ou grillagée (qui se déploie naturellement une fois sortie du cathéter 12) qui comporte au moins une pluralité de fils agencés pour former ensemble un maillage ou treillis. Dans cette première réalisation, le maillage présente des ouvertures (ou espaces inter-maille), entre les fils, dont les dimensions sont inférieures aux tailles des particules de sorte que ces particules ne puissent pas s'échapper de l'enveloppe expansible 13.

Dans une seconde réalisation, l'enveloppe expansible 13 est une enveloppe pleine (non maillée) réalisée dans un matériau à la fois biocompatible et élastique ou gonflable ; étant noté que ce matériau peut être résorbable ou non résorbable. Dans cette seconde réalisation, l'enveloppe expansible 13 est gonflée par injection de la composition liquide ou gel (telle que la colle biologique ou le gel polymérique précédemment décrits) via l'intérieur de l'organe de poussée tubulaire 14. La pression d'injection de la composition liquide ou gel va contribuer à déployer l'enveloppe expansible 13 à l'intérieur de la cavité osseuse 90. Une fois l'enveloppe expansible 13 gonflée, les particules sont injectées à l'intérieur de l'enveloppe expansible 13 en passant aussi à l'intérieur de l'organe de poussée tubulaire 14.

Une fois que l'enveloppe expansible 13 est remplie en particules (comme visible sur la Figure 4(f)), la bague 16 est fermée ou bouchée, et la bague 16 est détachée de l'organe de poussée tubulaire 14, permettant ainsi de retirer à la fois le cathéter 12 et l'organe de poussée tubulaire 14, pour laisser dans la cavité osseuse l'enveloppe expansible 13 remplie en particules et fermée par la bague 16 (comme visible sur la Figure 4(g)).

## Revendications

1. Dispositif d'injection (1) adapté pour une injection de particules à l'intérieur d'une cavité osseuse (90), ledit dispositif d'injection (1) comprenant :
- un réservoir (2) propre à contenir au moins en partie les particules ;
- une aiguille d'injection (3) de forme tubulaire autour d'un axe principal (AP) et munie d'une extrémité proximale (30) fixée sur une embase (20) du réservoir (2) et ouverte sur le réservoir (2), et d'une extrémité distale (31) opposée à l'extrémité proximale (30) et ouverte et **caractérisé en ce que** le dispositif comprend de plus
- une vis sans fin (4) mobile en rotation autour de l'axe principal (AP) à l'intérieur de l'aiguille d'injection (3) et s'étendant au moins depuis l'extrémité proximale (30) jusqu'à au moins l'extrémité distale (31) de l'aiguille d'injection (3) ; et
- un actionneur (40) couplé à la vis sans fin (4) pour l'entraîner en rotation.

2. Dispositif d'injection (1) selon la revendication 1, dans lequel l'actionneur (40) comprend un arbre (42) couplé en rotation à la vis sans fin (4), ledit arbre (42) sortant du réservoir (2) étant accouplé à un moteur (41).

3. Dispositif d'injection (1) selon l'une quelconque des revendications 1 et 2, dans lequel un piston (5) est monté coulissant dans le réservoir (2) et mobile en translation selon l'axe principal (AP).

4. Dispositif d'injection (1) selon l'une quelconque des revendications 1 à 3, comprenant au moins un moyen de dispersion (6 ; 71 ; 72 ; 8) propre à disperser et mettre en suspension les particules dans une composition liquide ou gel, par exemple de type colle biologique ou gel polymérique, à l'intérieur du réservoir (2).

5. Dispositif d'injection (1) selon la revendication 4, dans lequel le moyen de dispersion est choisi parmi les moyens suivants ou une combinaison de tout ou partie des moyens suivants :
- un système d'introduction (6) pour une introduction de la composition liquide ou gel à l'intérieur du réservoir (2), ledit système d'introduction (6) comprenant un moyen de mise en circulation (60) situé à l'extérieur du réservoir (2) et assurant une circulation de la composition liquide ou gel dans un ou plusieurs conduits d'introduction (61) reliés à un ou plusieurs points d'introduction (62) débouchant dans l'embase (20) du réservoir (2) ;
- un système vibrateur (71) pour appliquer une vibration sur le réservoir (2) ou sur l'aiguille d'injection (3) ;
- un système de brassage (72) pour brasser les particules en solution dans la composition liquide ou gel à l'intérieur du réservoir (2) ;
- un système oscillant (8) comprenant un piston (5) monté coulissant dans le réservoir (2) et mobile en translation selon l'axe principal (AP), et un actionneur oscillant (80) propre à imprimer un mouvement d'oscillation au piston (5) selon l'axe principal (AP).

6. Dispositif d'injection (1) selon l'une quelconque des revendications 1 à 5, dans lequel la vis sans fin (4) s'étend au-delà de l'extrémité proximale (30) de l'aiguille d'injection (3), de sorte que la vis sans fin (4) présente au moins deux parties successives comprenant :
- une partie inférieure (43) qui s'étend depuis l'extrémité proximale (30) jusqu'à l'extrémité distale (31) de l'aiguille d'injection (3) ;
- une partie supérieure (44) qui prolonge la partie inférieure (43) au-delà de l'extrémité proximale (30) et qui s'étend à l'intérieur du réservoir (2).

7. Dispositif d'injection (1) selon l'une quelconque des revendications 1 à 6, dans lequel la vis sans fin (4) est ajustable en translation le long de l'axe principal (AP).

8. Dispositif d'injection (1) selon l'une quelconque des revendications 1 à 7, comprenant en outre un implant expansible (11) comprenant au moins un cathéter (12) et une enveloppe expansible (13) configurable entre :
- un état contracté dans lequel l'enveloppe expansible (13) est contrainte radialement dans le cathéter (12) pour une délivrance de l'enveloppe expansible (13) à l'intérieur de la cavité osseuse (90), et
- un état déployé dans lequel l'enveloppe expansible (13) est étendue hors du cathéter (12) avec une configuration en forme de poche pour être remplie en particules.

9. Kit d'injection comprenant au moins :
- un dispositif d'injection (1) selon l'une quelconque des revendications 1 à 8 ; et
- des particules adaptées pour être injectées à l'intérieur d'une cavité osseuse (90) au moyen dudit dispositif d'injection (1).

10. Kit d'injection selon la revendication 9, comprenant en outre un vibrateur osseux propre à appliquer une vibration sur un os (9) durant une injection de la composition de ciment osseux (10) à l'intérieur d'une cavité osseuse (90) dudit os (9).

11. Kit d'injection selon l'une quelconque des revendications 9 et 10, dans lequel les particules sont choisies parmi des particules d'hydroxyapatite, d'acide poly(lactique-glycolique), d'amidon ou de chitosane.

12. Kit d'injection selon l'une quelconque des revendications 9 à 11, dans lequel les particules sont en solution dans une composition liquide ou gel pour former ensemble une composition de ciment osseux (10), où ladite composition liquide ou gel comporte un solvant liquide ou gel apte à diffuser à travers l'os.

13. Kit d'injection selon la revendication 12, dans lequel la composition liquide ou gel est un gel polymérique comprenant un polymère en solution dans un solvant, ledit polymère étant choisi parmi :
- le polyéthylène glycol, l'acide hyaluronique ou l'hydroxypropyl methyl cellulose en solution dans de l'eau ;
- l'éthylène alcool vinylique ou l'acétate de cellulose en solution dans le diméthylsulfoxyde ;
- l'éthyl cellulose en solution dans l'éthanol.

14. Kit d'injection selon la revendication 12, dans lequel la composition liquide ou gel est une colle biologique qui est choisie parmi les colles biologiques à base de :
- fibrinogène ou thrombine, le fibrinogène et la thrombine étant optionnellement en association avec de l'aprotinine,
- fibrine autologue,
- collagène, ou
- N-butyl-cyano acrylate.

15. Kit d'injection selon l'une quelconque des revendications 12 à 14, dans lequel la composition liquide ou gel comprend des fibres et/ou un agent radio-opaque, qui est par exemple une huile éthiodisée ou une poudre métallique micronisée choisie parmi les poudres de tantale, tungstène, baryum, bismuth, iode minéral, ou une solution de gadolinium d'acide éthylènediaminetétracétique.

## Patentansprüche

1. Injektionsvorrichtung (1), die für eine Injektion von Teilchen in das Innere eines Knochenhohlraums (90) geeignet ist, wobei die Injektionsvorrichtung (1) umfasst:
- einen Behälter (2), der geeignet ist, die Teilchen mindestens zum Teil zu enthalten;
- eine Injektionsnadel (3), die von rohrförmiger Form um eine Hauptachse (AP) und mit einem proximalen Ende (30), das an einer Basis (20) des Behälters (2) befestigt und am Behälter (2) offen ist, und einem dem proximalen Ende (30) gegenüberliegenden und offenen distalen Ende (31) versehen ist,
und **dadurch gekennzeichnet, dass** die Vorrichtung weiter umfasst
- eine Schnecke (4), die im Inneren der Injektionsnadel (3) um die Hauptachse (AP) drehbeweglich ist und sich mindestens vom proximalen Ende (30) bis mindestens zum distalen Ende (31) der Injektionsnadel (3) erstreckt; und
- einen Aktor (40), der mit der Schnecke (4) gekoppelt ist, um sie in Drehung zu versetzen.

2. Injektionsvorrichtung (1) nach Anspruch 1, wobei der Aktor (40) eine Welle (42) umfasst, die mit der Schnecke (4) drehgekoppelt ist, wobei die Welle (42), die aus dem Behälter (2) austritt, an einen Motor (41) angekoppelt ist.

3. Injektionsvorrichtung (1) nach einem der Ansprüche 1 und 2, wobei ein Kolben (5) gleitend im Behälter (2) montiert und entlang der Hauptachse (AP) verschiebebeweglich ist.

4. Injektionsvorrichtung (1) nach einem der Ansprüche 1 bis 3, die mindestens ein Dispersionsmittel (6; 71; 72; 8) umfasst, das geeignet ist, die Teilchen in einer flüssigen oder Gel-Zusammensetzung, zum Beispiel vom Typ eines biologischen Klebers oder Polymergels, im Inneren des Behälters (2) zu dispergieren und in Suspension zu bringen.

5. Injektionsvorrichtung (1) nach Anspruch 4, wobei das Dispersionsmittel aus den folgenden Mitteln oder einer Kombination aller oder eines Teils der folgenden Mittel ausgewählt ist:
- einem Einführsystem (6) für ein Einführen der flüssigen oder Gel-Zusammensetzung in das Innere des Behälters (2), wobei das Einführsystem (6) ein Umwälzmittel (60) umfasst, das sich außerhalb des Behälters (2) befindet und eine Umwälzung der flüssigen oder Gel-Zusammensetzung in einer oder mehreren Einführleitungen (61), die mit einem oder mehreren in die Basis (20) des Behälters (2) mündende Einführpunkten (62) verbunden sind, gewährleistet;
- einem Vibrationssystem (71), um eine Vibration an den Behälter (2) oder an die Injektionsnadel (3) anzulegen;
- einem Rührsystem (72), um die Teilchen in Lösung in der flüssigen oder Gel-Zusammensetzung im Inneren des Behälters (2) zu rühren;
- einem oszillierenden System (8), das einen Kolben (5), der gleitend im Behälter (2) montiert und entlang der Hauptachse (AP) verschiebebeweglich ist, und einen oszillierenden Aktor (80) umfasst, der geeignet ist, dem Kolben (5) entlang der Hauptachse (AP) eine Oszillationsbewegung zu verleihen.

6. Injektionsvorrichtung (1) nach einem der Ansprüche 1 bis 5, wobei sich die Schnecke (4) über das proximale Ende (30) der Injektionsnadel (3) hinaus erstreckt, sodass die Schnecke (4) mindestens zwei aufeinanderfolgende Teile aufweist, welche umfassen:
- einen unteren Teil (43), der sich vom proximalen Ende (30) bis zum distalen Ende (31) der Injektionsnadel (3) erstreckt;
- einen oberen Teil (44), der den unteren Teil (43) über das proximale Ende (30) hinaus verlängert und der sich in das Innere des Behälters (2) erstreckt.

7. Injektionsvorrichtung (1) nach einem der Ansprüche 1 bis 6, wobei die Schnecke (4) verschiebbar entlang der Hauptachse (AP) verstellbar ist.

8. Injektionsvorrichtung (1) nach einem der Ansprüche 1 bis 7, die weiter ein expandierbares Implantat (11) umfasst, das mindestens einen Katheter (12) und eine expandierbare Hülle (13) umfasst, die konfigurierbar ist zwischen:
- einem zusammengezogenen Zustand, in dem die expandierbare Hülle (13) für ein Einbringen der expandierbaren Hülle (13) in das Innere des Knochenhohlraums (90) radial im Katheter (12) eingespannt ist, und
- einem ausgezogenen Zustand, in dem die expandierbare Hülle (13) mit einer taschenförmigen Konfiguration aus dem Katheter (12) herausgebracht ist, um mit Teilchen gefüllt zu werden.

9. Injektionsset, das mindestens umfasst:
- eine Injektionsvorrichtung (1) nach einem der Ansprüche 1 bis 8; und
- Teilchen, die geeignet sind, mittels der Injektionsvorrichtung (1) in das Innere eines Knochenhohlraums (90) injiziert zu werden.

10. Injektionsset nach Anspruch 9, das weiter einen Knochenvibrator umfasst, der geeignet ist, während einer Injektion der Knochenzementzusammensetzung (10) in das Innere eines Knochenhohlraums (90) des Knochens (9) eine Vibration an einen Knochen (9) anzulegen.

11. Injektionsset nach einem der Ansprüche 9 und 10, wobei die Teilchen ausgewählt sind aus Hydroxylapatit-, Poly(milch-glycolsäure)-, Stärke- oder Chitosanteilchen.

12. Injektionsset nach einem der Ansprüche 9 bis 11, wobei sich die Teilchen in einer flüssigen oder Gel-Zusammensetzung in Lösung befinden, um zusammen eine Knochenzementzusammensetzung (10) zu bilden, wobei die flüssige oder Gel-Zusammensetzung ein flüssiges oder Gel-Lösungsmittel umfasst, das in der Lage ist, durch den Knochen zu diffundieren.

13. Injektionsset nach Anspruch 12, wobei die flüssige oder Gel-Zusammensetzung ein Polymer-Gel ist, das ein Polymer in Lösung in einem Lösungsmittel umfasst, wobei das Polymer ausgewählt ist aus:
- Polyethylenglykol, Hyaluronsäure oder Hydroxypropylmethylcellulose in Lösung in Wasser;
- Ethylenvinylalkohol oder Celluloseacetat in Lösung in Dimethylsulfoxid;
- Ethylcellulose in Lösung in Ethanol.

14. Injektionsset nach Anspruch 12, wobei die flüssige oder Gel-Zusammensetzung ein biologischer Kleber ist, der ausgewählt ist aus biologischen Klebern auf Basis von:
- Fibrinogen oder Thrombin, wobei das Fibrinogen und das Thrombin gegebenenfalls in Kombination mit Aprotinin vorliegen,
- autologem Fibrin,
- Kollagen, oder
- N-Butyl-Cyanoacrylat.

15. Injektionsset nach einem der Ansprüche 12 bis 14, wobei die flüssige oder Gel-Zusammensetzung Fasern und/oder ein strahlenundurchlässiges Mittel umfasst, das zum Beispiel ein ethiodisiertes Öl oder ein mikronisiertes Metallpulver ist, ausgewählt aus Tantal-, Wolfram-, Barium-, Wismut-, Jodmineralpulvern oder einer Ethylendiamintetraessigsäure-Gadolinium-Lösung.

## Claims

1. An injection device (1) adapted for injecting particles into a bone cavity (90), said injection device (1) comprising:
- a reservoir (2) capable of containing at least in part the particles;
- an injection needle (3) having a tubular shape around a main axis (AP) and provided with a proximal end (30) fixed to a base (20) of the reservoir (2) and open to the reservoir (2), and a distal end (31) opposite to the proximal end (30) and open;
and **characterized in that** the device further comprises :- a worm screw (4) movable in rotation around the main axis (AP) inside the injection needle (3) and extending at least from the proximal end (30) up to at least the distal end (31) of the injection needle (3); and
- an actuator (40) coupled to the worm screw (4) to drive it in rotation.

2. The injection device (1) according to claim 1, wherein the actuator (40) comprises a shaft (42) coupled in rotation to the worm screw (4), said shaft (42emerging from the reservoir (2) to be engaged with a motor (41).

3. The injection device (1) according to any one of claims 1 and 2, wherein a piston (5) is slidably mounted in the reservoir (2) and is movable in translation according to the main axis (AP).

4. The injection device (1) according to any one of claims 1 to 3, comprising at least one dispersion means (6; 71; 72; 8) capable of dispersing and suspending the particles in a liquid or gel composition, for example of the biological glue or polymeric gel type, inside the reservoir (2).

5. The injection device (1) according to claim 4, wherein the dispersion means is selected from among the following means or a combination of all or part of the following means:
- an introduction system (6) for introducing the liquid or gel composition inside the reservoir (2), said introduction system (6) comprising a circulation means (60) located outside the reservoir (2) and ensuring a circulation of the liquid or gel composition in one or several introduction conduits (61) connected to one or several introduction points (62) opening into the base (20) of the reservoir (2);
- a vibrator system (71) for applying a vibration to the reservoir (2) or to the injection needle (3):
- a stirring system (72) for stirring the particles dissolved in the liquid or gel composition inside of the reservoir (2);
- an oscillating system (8) comprising a piston (5) slidably mounted in the reservoir (2) and movable in translation according to the main axis (AP), and an oscillating actuator (80) capable of imparting an oscillating movement to the piston (5) according to the main axis (AP).

6. The injection device (1) according to any one of claims 1 to 5, wherein the worm screw (4) extends beyond the proximal end (30) of the injection needle (3), such that the worm screw (4) has at least two successive portions comprising:
- a lower portion (43) which extends from the proximal end (30) up to the distal end (31) of the injection needle (3);
- an upper portion (44) which prolongs the lower portion (43) beyond the proximal end (30) and which extends inside the reservoir (2).

7. The injection device (1) according to any one of claims 1 to 6, wherein the worm screw (4) is adjustable in translation along the main axis (AP).

8. The injection device (1) according to any one of claims 1 to 7, further comprising an expandable implant (11) comprising at least one catheter (12) and an expandable envelope (13) that may be configured between:
- a contracted state in which the expandable envelope (13) is radially constrained in the catheter (12) for delivery of the expandable envelope (13) within the bone cavity (90), and
- a deployed state in which the expandable envelope (13) is extended out of the catheter (12) with a bag-like configuration to be filled with particles.

9. An injection kit comprising at least:
- an injection device (1) according to any one of claims 1 to 8; and
- particles adapted to be injected inside a bone cavity (90) by means of said injection device (1).

10. The injection kit according to claim 9, further comprising a bone vibrator capable of applying a vibration to a bone (9) during an injection of the bone cement composition (10) into a bone cavity (90) of said bone (9).

11. The injection kit according to any one of claims 9 and 10, wherein the particles are selected from particles of hydroxyapatite, poly(lactic-glycolic)acid, starch or chitosan.

12. The injection kit according to any one of claims 9 to 11, wherein the particles are dissolved in a liquid or gel composition to form together a bone cement composition (10), wherein said liquid or gel composition includes a liquid or gel solvent adapted to diffuse through the bone.

13. The injection kit according to claim 12, wherein the liquid or gel composition is a polymeric gel comprising a polymer dissolved in a solvent, said polymer being selected from:
- polyethylene glycol, hyaluronic acid or hydroxypropyl methylcellulose dissolved in water:
- ethylene vinyl alcohol or cellulose acetate dissolved in dimethyl sulfoxide;
- ethyl cellulose dissolved in ethanol.

14. The injection kit according to claim 12, wherein the liquid or gel composition is a biological glue which is selected from biological glues based on:
- fibrinogen or thrombin, fibrinogen and thrombin being optionally in association with aprotinin,
- autologous fibrin,
- collagen, or
- N-butyl-cyanoacrylate.

15. The injection kit according to any one of claims 12 to 14, wherein the liquid or gel composition comprises a radiopaque agent, which is for example an ethiodized oil or a micronized metal powder selected from powders of tantalum, tungsten, barium, bismuth, mineral iodine, or a gadolinium solution of ethylenediaminetetraacetic acid.
